# EUROPEAN PATENT APPLICATION

(11) **EP 2 878 285 A1**
(43) Date of publication of application: **03.06.2015**
(21) Application number: 13195184.0
(22) Date of filing: 29.11.2013
(51) Int. Cl.: A61F 5/055

(54) **A therapeutic device for neck pain**

(71) Applicant: University of Limerick, Co. Limerick Limerick (IE)
(72) Inventor: Van De Ven, Pepijn, Newport, County Tipperary (IE); Cussen, John, Limerick (IE); Clifford, Amanda, Annacotty County Limerick (IE); Nelson, John, Limerick (IE); O'Riordan, Cliona, Macroom, County Cork (IE)
(74) Representative: Weldon, Michael James

(57) **Abstract**

A therapeutic device (1) comprises a body (2) with a shoulder -engaging part (12) and a chest-engaging part (11) and , elevated pivot supports (12) extending upwardly from the shoulder-engaging part (12) at locations to be on opposed sides of a patient's head in use. A head support (3) with a chin cradle (35) is pivotally connected by pivot joints (20) to the pivot supports (12). Torsion springs (24) bias the head support (3) upwardly. Sensors wirelessly provide data to a processor such as a smartphone so that the extent of movement of the head support can be monitored.

## Description

The invention relates to a device to strengthen neck muscles in people with weak neck muscles due to neck pain, following whiplash or the like injuries.

Current treatment of people with chronic neck pain consists of physiotherapy sessions in some cases augmented by the use of biofeedback devices such as the biostabilizer feedback used to strengthen the neck musculature. Due to the often infrequent contact with the physiotherapist these treatments can be suboptimal. Patients greatly profit from performing exercises at home at a more frequent basis, but the problem with these exercises is that they need to be performed correctly and regularly in order to yield good results.

An objective is to provide a device which is comfortable to wear, with minimal effect on patient mobility, and is more ergonomic than the prior art devices. Another objective is to achieve/ improve patient analysis.

### Summary of the Invention

According to the invention, there is provided a therapeutic device comprising:
a body with a shoulder-engaging part, a chest-engaging part, and elevated pivot supports extending upwardly from the shoulder-engaging part at locations to be on opposed sides of a patient's head in use,
a head support with a chin cradle and being pivotally connected by pivot joints to the pivot supports, and
bias means for biasing the head support upwardly.

In one embodiment, the head support comprises a pair of cranked arms, each having an outer part engaging the chin cradle and an inner part connected to one of said pivot joints. In one embodiment, the crank angle is in the range of 0° to 30°.

In one embodiment, the chin cradle is telescopically connected to the arms for adjustment of distance of the chin cradle from the pivot joints.

In one embodiment, the pivot joints form a pivot axis which is separated from the shoulder-engaging body part lower surface by a distance in the range of 40 mm to 70 mm, whereby the device is suitable for child use.

In one embodiment, the pivot joints form a pivot axis which is separated from the shoulder-engaging body part lower surface by a distance in the range of 70 mm to 100 mm, whereby the device is suitable for adult use.

In one embodiment, the head support has a default home position at which the chin cradle level is in the range of 20 mm to 36mm below the level of a pivot axis formed by the pivot joints as measured in use.

In one embodiment, the front of the chin cradle is in the range of 105 mm to 165 mm in front of a pivot axis formed by the pivot joints as viewed in the horizontal direction in use.

In one embodiment, the bias means is incorporated in the pivot joints.

In one embodiment, the bias means comprises torsion springs within the pivot joints. In one embodiment, the bias means includes magnetic brakes. In one embodiment, the bias means is user-adjustable to adjust torsion force applied.

In one embodiment, the bias means comprises a torsion spring mounted within each pivot joint between the body and a cap, and the cap is adjustable by rotation to a selected position in an action which twists the torsion spring to adjust applied torsion force.

In one embodiment, the cap is adapted to be engaged and disengaged with a gear on the body by pushing and pulling actions.

In one embodiment, each pivot joint comprises a return spring to provide a default engaged position for the cap.

In one embodiment, the sensors and/or a data processor are wired or wirelessly connected to a receiving processing device, and said processing device is adapted to generate outputs arising from monitoring of the movement of the head support and/or to relay data to a remote site for remote monitoring and feedback.,

In one embodiment, the sensors are mounted within the pivot joints.

In one embodiment, the device comprises a sensor arranged to detect force applied by a patient's chin against the chin cradle. In one embodiment, the sensors are wirelessly connected to the data processor.

Preferably, the device comprises at least one sensor for detecting applied bias to the head support. In one embodiment, said sensor is arranged to detect twisting of a torsion spring. In one embodiment, sensor is arranged to detect the mutual position of a cap to which one end of the spring is connected and the body.

### Detailed Description of the Invention

The invention will be more clearly understood from the following description of some embodiments thereof, given by way of example only with reference to the accompanying drawings in which:-
Fig. 1 is a side view showing a therapeutic device of the invention in use;
Fig. 2 is a perspective view from above of a therapeutic device of the invention in the configuration at which it would be worn by a patient;
Fig. 3 is a side view from the right side;
Fig. 4 is a plan view, with a head support bracket pivoted away from its in-use position;
Fig. 5 is a perspective view of the device;
Fig. 6 is a perspective view showing a pivot joint in more detail;
Fig. 7 is a cross-sectional view through the pivot joint; and
Fig. 8 is a view showing a rotary sensor which is used as part of a pivot joint.

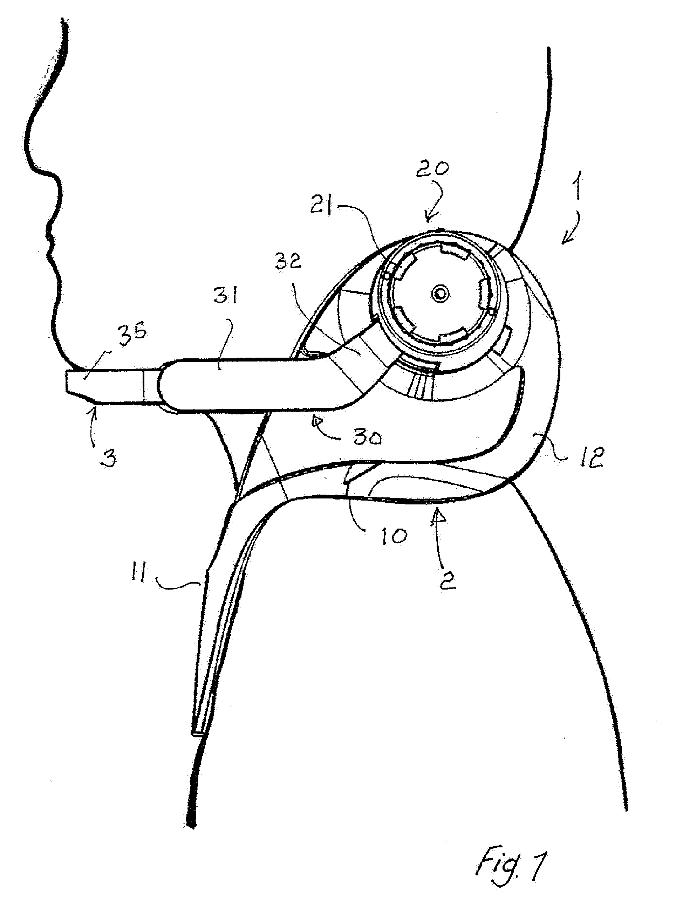

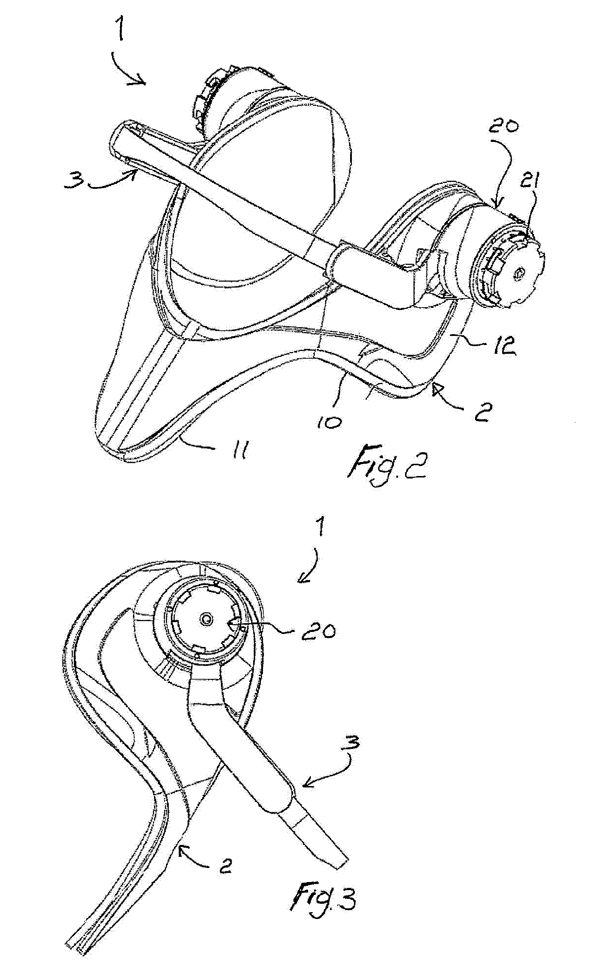

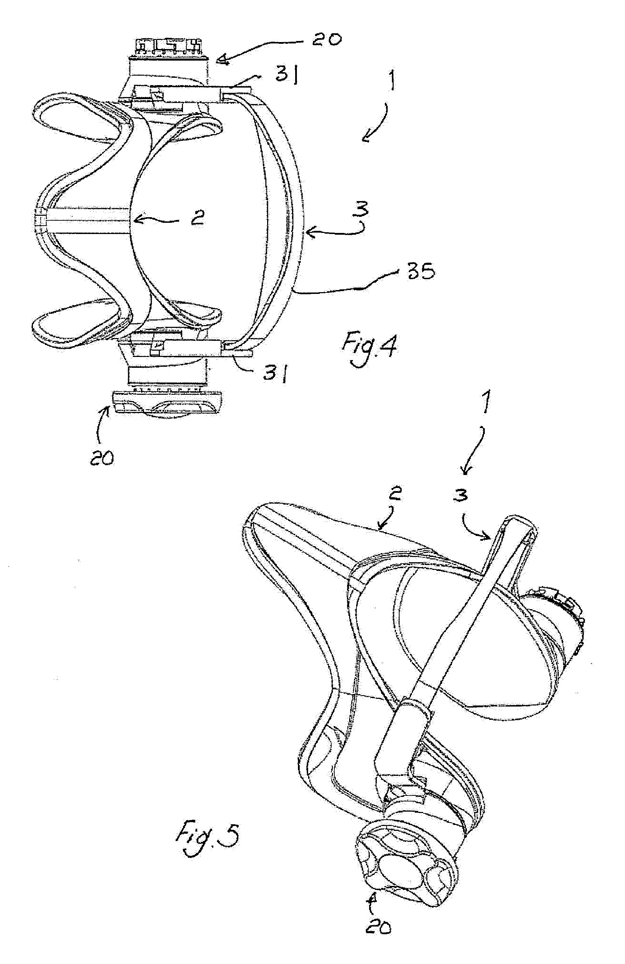

Referring to Figs. 1 to 8 a therapeutic device 1 of the invention is for assisting a patient to recover from whiplash or other neck injury. It is particularly suited to treatment of chronic neck injury.

The device 1 comprises a main body 2 and a head support bracket 3 pivotally connected to the body 2. The body 2 is of moulded plastics material, shaped to comfortably rest on a patient's shoulders and top of the chest. It has a part 10 for resting on the shoulders, and a lower depending part 11 for resting against the top of the chest for balanced, comfortable, support. The body 2 also comprises a pivot support 12 which extends upwardly from the shoulder part 10 on opposed sides of the patient's head in use.

The bracket 3 rotates about pivot joints 20 on the pivot supports 12 at locations, in use, in the regions of a patient's ears. In general, the distance of the axis formed by the opposed pivot joints 20 above the level of the shoulder-engaging part 10 lower surface is in the range of 40 mm to 70 mm for children and 70 mm to 100m for adults.

The head support bracket 3 comprises a chin cradle 35 connected on each lateral side to an arm 30, which is in turn pivotally connected to the pivot joints 20. Each arm 30 has an outer part 31 connected to an end of the chin support 25, and an inner part 32 connected to a pivot joint 20. The arm parts 31 and 32 are at an angle of about 25° to each other. The chin cradle 35 is telescopically connected to the arms 30, for adjustment. The arms 30 are parallel to each other. They move from a stationary 0° to a maximum collapse of 30°. The crank in each arm 30 allows convenient and ergonomic telescopic inward-outward adjustment of the position of the chin cradle 35. It also provides comfort for the user as the outer arm part 31 is largely below the level of the jaw bone.

The crank in the arms 30 also achieves excellent compatibility between the device 1 and the anatomy of the patient. The design of the arms allow for a flexion with rotation movement as in cranio-cervical flexion (flattening of the cervical lordosis/"chin to chest" movement occurring in the upper cervical cranio-cervical junction) which is imperative for proper exercise execution. As shown most clearly in Fig. 1 the crank in the arms 30 allows the level of the chin cradle 35 to be below the pivot axis to an extent in the range of 20 mm to 28 mm for children and 28 mm to 36mm for adults.

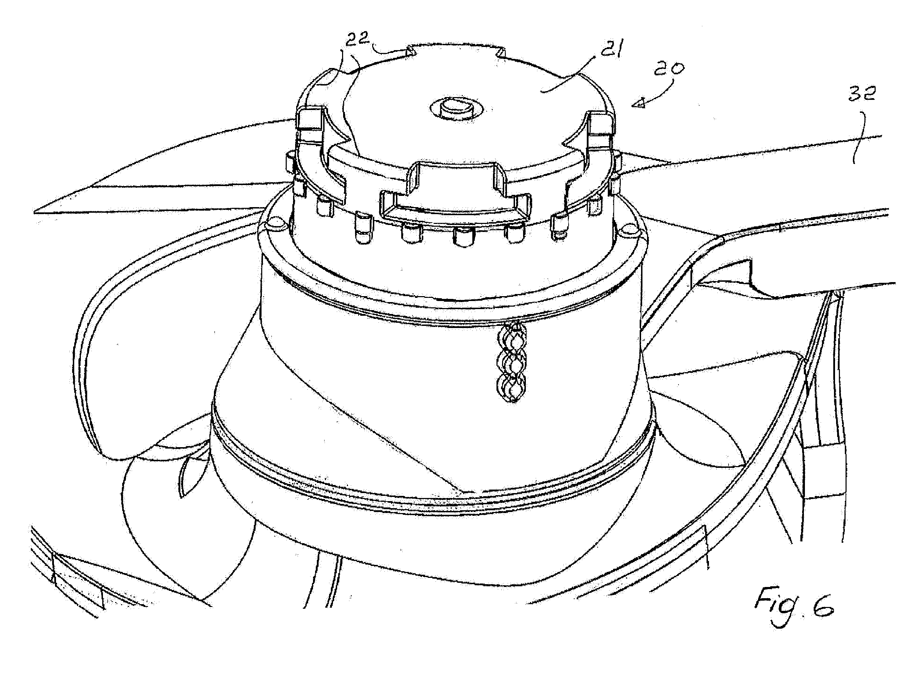

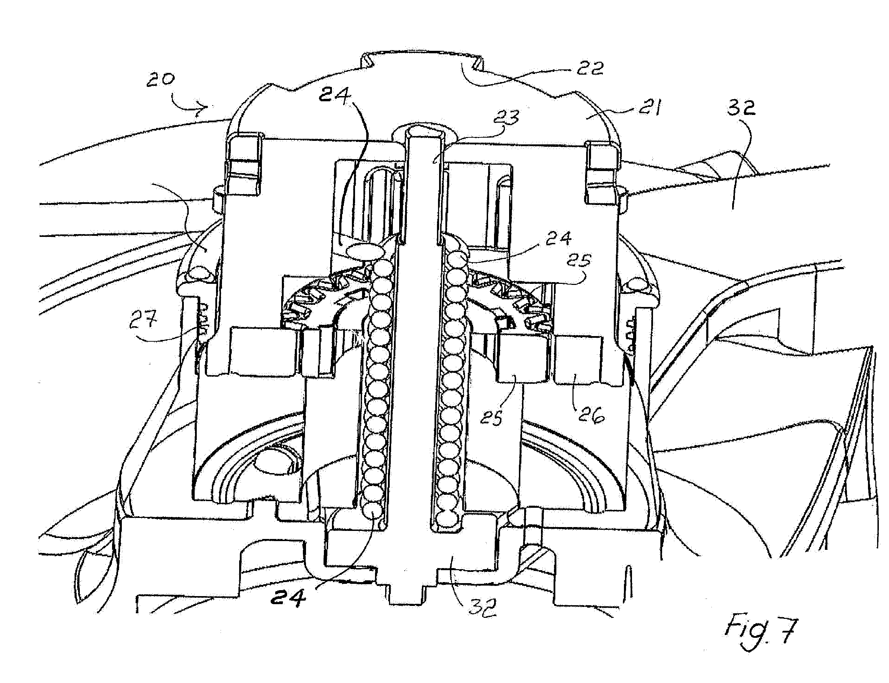

Referring to Figs. 6 and 7 each pivot joint 20 has a cap 21 which is rotatable with assistance of finger grips 22 about a pin 23 which is an integral extension of the arm 30. A helical spring 24 is anchored at its inner end on the inner arm part 32 and at its outer end on the cap 21. The cap 21 has an annular gear 26 with inward-facing teeth and is pressed outwardly by a return spring (not shown) to a position where it engages a gear 25 fixed to the body 2.

Hence, in the position shown in Fig. 7 the torsion spring 24 provides bias against downward movement of the chin cradle 35 by virtue of being anchored at one end on the cap 21 and at the other to the arm 30. The level of bias can be adjusted by the user pushing the cap 21 in against the return spring so that the gear 26 disengages from the gear 25, and he may then rotate the cap 21 to wind or unwind the spring 24 to increase or decrease the torque respectively.

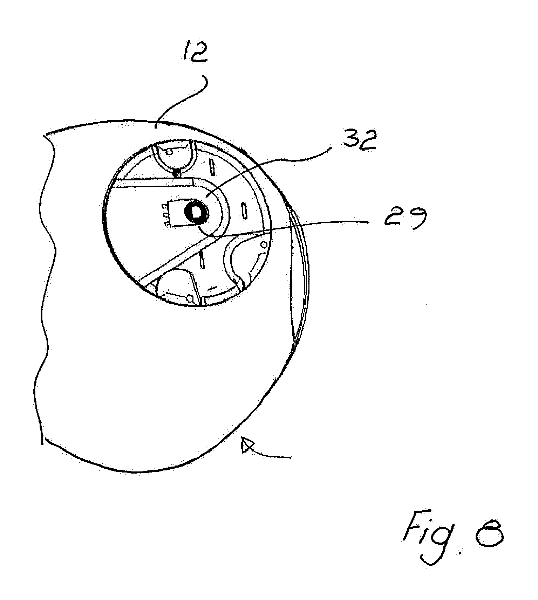

The extent of rotation of the head support 3 is sensed by a potentiometer sensor 29 mounted between the body 2 below the pin 23 and the base of the pin 23 (which is on the arm 30). This is shown most clearly in Fig. 8. However, it will be appreciated that a variety of other types of sensors could be used for detecting mutual rotation. Importantly the mechanical arrangement provides a basis for gathering a lot of very useful information by simply detecting relative rotation of two parts with very low rotational speeds. Likewise the

The sensors are physically linked to signal processing circuits which are wirelessly linked to a processing device. The latter may in some embodiments be a smartphone programmed with a suitable application. The communication protocol may be Bluetooth.

The signal processing circuit comprises a dedicated circuit board with an MSP430 microprocessor is used to sample, digitise and process the data originating from the sensors. The hardware further consists of a third party Bluetooth radio circuit which is used to communicate sensor information to a base station (currently a Bluetooth enabled mobile phone).

The signal processing circuit software consists of code for executing sampling, storage and communication algorithms running on the MSP430. Software on the mobile phone consists of a Bluetooth interface for communication with the sensor and a graphical user interface that allows the user to view data from the device and calibrate the device.

It will be appreciated that the invention offers a solution for the currently suboptimal treatment of chronic neck pain. This is done by allowing the user to safely perform exercises to improve deep neck flexor strength whilst being monitored by sensors on the device. The device guarantees the correct execution of the exercises and the monitoring functionality enables the user and other stake holders to monitor that the exercise regime is being followed.

The spring-loaded bracket 3 sits under the user's chin and is mounted in the device in such a way that the user can push down on the bracket and pivot the latter towards their chest. Normal range of rotation is from 0° in the 'neutral' position to around 17° in a healthy population. The device allows a rotation of about 30°. In doing this, the springs 24 offer a resistance to the motion which results in an exercising effect on the user's neck muscles. Due to the particular motion enforced by the device's design, the deep-neck flexors in particular are exercised.

The movement of the bracket 3 is monitored using an angular sensor and force sensors 29. The information coming from these sensors is used for feedback to the user and their therapist and can be used for review of the exercises done, or as motivational information for the user.

The invention gives the patient complete freedom to perform the designated exercise program in the position and environment they feel most comfortable in. This is crucial for adherence to the exercise regime and is a major improvement over prior approaches such as therapy while the patient is lying down.

As mentioned above, the device may include data collection and processing capability, and so the exercise execution can be monitored using a suite of sensors. In addition to the bracket's angle of rotation being recorded using an angular sensor, force exerted by the user on the chin cradle 35 can be measured directly using force or load sensors or strain gauges.

The resistive angular sensor 29 is mounted to the device body and measures the rotation of the bracket 3 axis. This signal is sampled and used for two purposes:
1. The sensor output provides direct angular information on the exercise performed. For proper exercise execution it is important to bend the chin towards the chest over a range specified by the physiotherapist.
2. The angular information can be used indirectly to obtain the force with which the user pushes down on the chin bracket. This information is obtained by calculating the extension of the spring based on the rotation of the bracket 3 (with which the spring is connected) which results in the force exerted on the spring. By applying moment equations, the force exerted by the user can then be obtained. This approach may include sensors to detect the twisting/winding extent of the spring 24, by for example detecting mutual position of the cap 21 and the body 3, which can be detected by similar potentiometer-based sensors mounted in the cap 21.

As noted above, a force sensor can be provided in the chin cradle 35, located under the user's chin to directly measure the force exerted by the user. Such load sensors are well known.

The use of the device as a remotely monitored home exercise device will result in improved treatment outcomes and significant cost savings to the customer. It may for example allow physiotherapists to treat more patients concurrently thus increasing productivity and revenue. Using dedicated software, the physiotherapist is able to view exactly what exercises their patient is doing and when and how effectively they are performed. As the software will automatically generate alerts when exercise adherence is not satisfactory, this may optimise both self-management and the supervisory role of the physiotherapist. The extent of data which can be provided by the sensors allows a wide variety and extent of algorithms for the data processing device, thereby allowing direct feedback to the patient or via a remote linked to a central site where a qualified clinician may provide additional input and monitoring.

The following lists some advantages:
- Shorter recovery time.
- Monitoring exercise adherence and execution automatically.
- Direct feedback to the user, thus increasing the quality of exercise execution, exercise adherence and patient motivation.
- Variable exercise resistance, which results in a more suitable build-up of exercise intensity.
- Objective feedback on exercise execution, which is a good measure for patient progression.
- The product will enable physiotherapists to increase productivity and thus revenue.

The invention is not limited to the embodiments described but may be varied in construction and detail. For example, the head support bias may alternatively be provided by a different bias means such as a spring or elastic member acting in a linear manner on the arms, or indeed any suitable electrical or electro-magnetic mechanism. The data processor (MSP430) which is local on the head support or body may be wired or wirelessly connected to a separate receiving device such as a smartphone.

## Claims

1. A therapeutic device (1) comprising:
a body (2) with a shoulder-engaging part (12), a chest-engaging part (11), and elevated pivot supports (12) extending upwardly from the shoulder-engaging part (12) at locations to be on opposed sides of a patient's head in use,
a head support (3) with a chin cradle (35) and being pivotally connected by pivot joints (20) to the pivot supports (12), and
bias means (24) for biasing the head support(3) upwardly,
wherein the bias means is incorporated in the pivot joints (20).

2. A therapeutic device as claimed in claim 1, wherein the head support (3) comprises a pair of cranked arms (30), each having an outer part (31) engaging the chin cradle (35) and an inner part (32) connected to one of said pivot joints (20).

3. A therapeutic device as claimed in claims 1 or 2, wherein the chin cradle (35) is telescopically connected to the arms (30) for adjustment of distance of the chin cradle (35) from the pivot joints (20).

4. A therapeutic device as claimed in claims 1 or 2 or 3, wherein the bias means comprises torsion springs (24) within the pivot joints.

5. A therapeutic device as claimed in any preceding claim, wherein the bias means is user-adjustable to adjust torsion force applied.

6. A therapeutic device as claimed in claim 5, wherein the bias means comprises a torsion spring (24) mounted within each pivot joint (20) between the body and a cap, and the cap (21) is adjustable by rotation to a selected position in an action which twists the torsion spring to adjust applied torsion force.

7. A therapeutic device as claimed in claim 6, wherein the cap is adapted to be engaged and disengaged with a gear on the body by pushing and pulling actions.

8. A therapeutic device as claimed in claim 7, wherein each pivot joint comprises a return spring to provide a default engaged position for the cap (21).

9. A therapeutic device as claimed in any preceding claim, wherein the device further comprises sensors (29) to detect movement of the head support (3), and a data processor for receiving and processing said signals to provide an output.

10. A therapeutic device as claimed in claim 9, wherein the sensors are mounted within the pivot joints.

11. A therapeutic device as claimed in any preceding claim, wherein the device comprises a sensor arranged to detect force applied by a patient's chin against the chin cradle (35).

12. A therapeutic device as claimed in any of claims 9, 10, or 11, wherein the sensors and/or a data processor are wired or wirelessly connected to a receiving processing device, and said processing device is adapted to generate outputs arising from monitoring of the movement of the head support and/or to relay data to a remote site for remote monitoring and feedback.,

13. A therapeutic device as claimed in any preceding claim, comprising at least one sensor for detecting applied bias to the head support.

14. A therapeutic device as claimed in claim 13, wherein said sensor is arranged to detect twisting of a torsion spring.

15. A therapeutic device as claimed in claim 14, wherein sensor is arranged to detect the mutual position of a cap (21) to which one end of the spring (24) is connected, and the body (2).
